(19) Europäisches Patentamt
European Patent Office
Office européen des brevets

(11) Veröffentlichungsnummer: **0 518 215 A1**

(12) **EUROPÄISCHE PATENTANMELDUNG**

(21) Anmeldenummer: **92109469.4**

(51) Int. Cl.5: **C07C 381/14**, A01N 47/42

(22) Anmeldetag: **04.06.92**

(30) Priorität: **08.06.91 DE 4118942**

(43) Veröffentlichungstag der Anmeldung:
**16.12.92 Patentblatt 92/51**

(84) Benannte Vertragsstaaten:
**PT**

(71) Anmelder: **HOECHST AKTIENGESELLSCHAFT**
**Postfach 80 03 20**
**W-6230 Frankfurt am Main 80(DE)**

(72) Erfinder: **Mildenberger, Hilmar, Dr.**
**Fasanenstrasse 24**
**W-6233 Kelkheim(DE)**
Erfinder: **Zurmühlen, Frank, Dr.**
**Inselsbergstrasse 13**
**W-6230 Frankfurt am Main 80(DE)**
Erfinder: **Braun, Peter, Dr.**
**Pfarrer-Dorn-Strasse 13**
**W-6500 Mainz(DE)**
Erfinder: **Sachse, Burkhard, Dr.**
**An der Ziegelei 30**
**W-6233 Kelkheim(DE)**

(54) **Sulfonylformamidrazone, Verfahren zu ihrer Herstellung, sie enthaltende Mittel und ihre Verwendung gegen Pflanzenschädlinge.**

(57) Die Erfindung betrifft Sulfonylformamidrazone der allgemeinen Formel (I) und deren Tautomere der allgemeinen Formel (II),

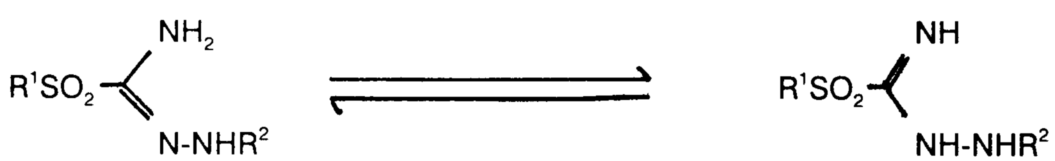

(I) (II),

worin $R^1$ gegebenenfalls substituiertes Alkyl, Alkenyl, Alkinyl, Cycloalkyl, Cycloalkenyl, Phenyl oder einen wie in der Beschreibung definierten Rest der Formel $-A-NR^2_2$, $-A-CO-R^4$, $-A-SO_2R^4$, $-A-SOR^4$, $-A-SR^4$ oder $-S-P(O)R^4_2$ bedeutet und $R^2$ für gegebenenfalls substituiertes Alkyl, Alkenyl, Alkinyl, Phenyl, Benzyl oder einen wie in der Beschreibung definierten Rest der Formel $-CO-R^2$ oder $-SO_2R^2$ steht. Die Erfindung betrifft weiterhin ein Verfahren zu deren Herstellung, diese enthaltende Mittel sowie deren Verwendung zur Bekämpfung von Pflanzenschädlingen und als Insektizid.

EP 0 518 215 A1

Die vorliegende Erfindung betrifft Sulfonylformamidrazone, Verfahren zu ihrer Herstellung, sie enthaltende Mittel sowie ihre Verwendung gegen Pflanzenschädlinge, insbesondere als Fungizide.

Gegenstand der Erfindung sind Sulfonylformamidrazone der allgemeinen Formel (I) und deren Tautomere der allgemeinen Formel (II),

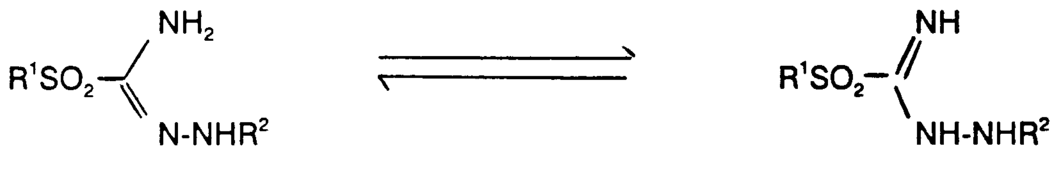

(I) (II),

worin

$R^1$ = Alkyl, Alkenyl, Alkinyl, Cycloalkyl, Cycloalkenyl, wobei die 5 Gruppen unabhängig voneinander unsubstituiert oder ein- oder mehrfach durch die Reste Halogen, Cyano, Nitro, Mono- und Dialkylamino, Alkoxy, Alkenyloxy, Alkinyloxy, Alkylthio, Alkenylthio, Alkinylthio substituiert sein können oder einen Rest der Formel

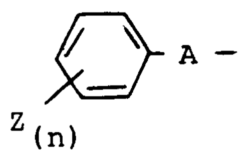

,

$R^2$ = Alkyl, Alkenyl, Alkinyl, Phenyl und Benzyl, wobei die 5 Gruppen unabhängig voneinander unsubstituiert oder ein- oder mehrfach durch die Reste Halogen, Cyano, Nitro, Mono- und Dialkylamino, Alkoxy, Haloalkyl substituiert sein können oder einen Rest der Formel $-C(O)-R^{2'}$, $-SO_2R^{2'}$, worin

$R^{2'}$ = Alkyl, Alkenyl, Alkinyl, Phenyl und Benzyl, wobei die 5 Gruppen unabhängig voneinander unsubstituiert oder ein- oder mehrfach durch die Reste Halogen, Cyano, Nitro, Mono- und Dialkylamino, Alkoxy, Haloalkyl substituiert sein können, bedeutet,

n = eine ganze Zahl von 0 - 5,

A = eine direkte Bindung, gesättigte oder ungesättigte, verzweigte oder unverzweigte Alkylkette,

Z = Wasserstoff, Halogen, Nitro, Cyano, Alkyl, Alkenyl, Alkinyl, Cycloalkyl, Cycloalkenyl, Alkoxy, Alkenyloxy, Alkinyloxy, Phenyl, Phenoxy, Haloalkyl, Haloalkoxy, Alkylthio, Haloalkylthio oder einen Rest der Formel
$-A-NR^3_2$ bedeuten, worin

$R^3$ = unabhängig voneinander Alkyl, Alkenyl, Alkinyl, Cycloalkyl, Phenyl, Benzyl, Alkoxy, Alkenyloxy, Alkinyloxy, Phenyloxy, Benzyloxy, Alkoxyalkyl, Alkylthioalkyl, Haloalkoxyalkyl, Haloalkylthioalkyl, Haloalkyl, Wasserstoff oder einen Rest der Formel
$-C(O)R^4$, $-SO_2R^4$, $-SOR^4$, $SR^4$ oder

$NR^3_2$ = Morpholino, Dialkylmorpholino, Piperidino, Piperazino, Hydrazino, Hydroxylamino, Phthalimido, Maleinimido, Dihydrophthalimido oder einen Rest der Formel
$-A-C(O)R^4$ bedeutet, worin

$R^4$ = Wasserstoff, Alkyl, Alkenyl, Alkinyl, Cycloalkyl, Phenyl, Phenoxy, Phenylthio, OH, SH, Alkoxy, Alkenyloxy, Alkinyloxy, Alkylthio, Alkenylthio, Alkinylthio, Cycloalkylthio, Cycloalkoxy, Haloalkoxy, Haloalkylthio, Alkoxyalkyl, Alkylthioalkyl oder
$-NR^5_2$ bedeutet, worin

$R^5$ = unabhängig voneinander Alkyl, Alkenyl, Alkinyl, Cycloalkyl, Phenyl, Benzyl, Wasserstoff, Alkoxyalkyl, Alkylthioalkyl oder

$NR^5_2$ = Piperidino, Morpholino, Piperazino, Hydrazino oder Hydroxylamino oder einen Rest der Formel
$-A-SO_2R^4$, $-A-SOR^4$, $-A-SR^4$, $-A-P(O)R^4_2$ bedeutet.

Bevorzugt sind Verbindungen der Formel (I) und (II), in denen

$R^1$ = $(C_1-C_6)$-Alkyl, $(C_2-C_4)$-Alkenyl, $(C_2-C_4)$-Alkinyl, $(C_3-C_6)$-Cycloalkyl, $(C_3-C_6)$-Cycloalkenyl, wobei die 5 Gruppen unabhängig voneinander unsubstituiert oder ein- oder mehrfach durch die Reste Halogen, Cyano, Nitro, Mono- und Di-$(C_1-C_6)$-alkylamino, $(C_1-C_4)$-Alkoxy, $(C_2-C_4)$-Alkenyloxy, $(C_2-C_4)$-Alkinyloxy, $(C_1-C_4)$-Alkylthio, $(C_2-C_4)$-Alkenylthio, $(C_2-C_4)$-Alkinylthio substituiert sein können oder einen Rest der Formel

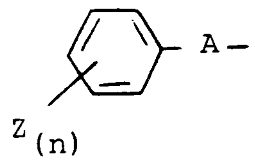

,

$R^2$ = $(C_1-C_6)$-Alkyl, $(C_2-C_4)$-Alkenyl, $(C_2-C_4)$-Alkinyl, Phenyl und Benzyl, wobei die 5 Gruppen unabhängig voneinander unsubstituiert oder ein- oder mehrfach durch die Reste Halogen, Cyano, Nitro, Mono- und Di-$(C_1-C_4)$-alkylamino, $(C_1-C_4)$-Alkoxy, Halo-$(C_1-C_4)$-alkyl substituiert sein können oder einen Rest der Formel
$-C(O)R^{2'}$, $-SO_2R^{2'}$, worin

$R^{2'}$ = $(C_1-C_6)$-Alkyl, $(C_2-C_4)$-Alkenyl, $C_2-C_4)$-Alkinyl, Phenyl und Benzyl, wobei die 5 Gruppen unabhängig voneinander unsubstituiert oder ein- oder mehrfach durch die Reste Halogen, Cyano, Nitro, Mono- und Di-$(C_1-C_4)$-alkylamino, $(C_1-C_4)$-Alkoxy, Halo-$(C_1-C_4)$-alkyl substituiert sein können, bedeutet,

n = eine ganze Zahl von 0 - 5,

A = eine direkte Bindung, gesättigte oder ungesättigte, verzweigte oder unverzweigte Alkylkette,

Z = Wasserstoff, Halogen, Nitro, Cyano, $(C_1-C_6)$-Alkyl, $(C_2-C_4)$-Alkenyl, $(C_2-C_4)$-Alkinyl, $(C_3-C_6)$-Cycloalkyl, $(C_3-C_6)$-Cycloalkenyl, $(C_1-C_4)$-Alkoxy, $(C_2-C_4)$-Alkenyloxy, $(C_2-C_4)$-Alkinyloxy, Phenyl, Phenoxy, Halo-$(C_1-C_4)$-alkyl, Halo-$(C_1-C_4)$-alkoxy, $(C_1-C_4)$-Alkylthio, Halo-$(C_1-C_4)$-alkylthio oder einen Rest der Formel
$-A-NR^3{}_2$ bedeuten, worin

$R^3$ = unabhängig voneinander $(C_1-C_6)$-Alkyl, $(C_2-C_4)$-Alkenyl, $(C_2-C_4)$-Alkinyl, $(C_3-C_6)$-Cycloalkyl, Phenyl, Benzyl, $(C_1-C_4)$-Alkoxy, $(C_2-C_4)$-Alkenyloxy, $(C_2-C_4)$-Alkinyloxy, Phenyloxy, Benzyloxy, $(C_1-C_4)$-Alkoxy-$(C_1-C_4)$-alkyl, $(C_1-C_4)$-Alkylthio-$(C_1-C_4)$-alkyl, Halo-$(C_1-C_4)$-alkoxy-$(C_1-C_4)$-alkyl, Halo-$(C_1-C_4)$-alkylthio-$(C_1-C_4)$-alkyl, Halo-$(C_1-C_4)$-alkyl, Wasserstoff oder einen Rest der Formel
$-C(O)R^4$, $-SO_2R^4$, $-SOR^4$, $SR^4$ oder

$NR^3{}_2$ = Morpholino, Dialkylmorpholino, Piperidino, Piperazino, Hydrazino, Hydroxylamino, Phthalimido, Maleinimido, Dihydrophthalimido oder einen Rest der Formel
$-A-C(O)R^4$ bedeutet, worin

$R^4$ = Wasserstoff, $(C_1-C_6)$-Alkyl, $(C_2-C_4)$-Alkenyl, $(C_2-C_4)$-Alkinyl, $(C_3-C_6)$-Cycloalkyl, Phenyl, Phenoxy, Phenylthio, OH, SH, $(C_1-C_4)$-Alkoxy, $(C_2-C_4)$-Alkyloxy, $(C_2-C_4)$-Alkinyloxy, $(C_1-C_4)$-Alkylthio, $(C_2-C_4)$-Alkenylthio, $(C_2-C_4)$-Alkinylthio, $(C_3-C_6)$-Cycloalkylthio, $(C_3-C_6)$-Cycloalkyloxy, Halo-$(C_1-C_4)$-alkoxy, Halo-$(C_1-C_4)$-alkylthio, $(C_1-C_4)$-Alkoxy-$(C_1-C_4)$-alkyl, $(C_1-C_4)$-Alkylthio-$(C_1-C_4)$-alkyl, $-NR^5{}_2$ bedeutet, worin

$R^5$ = unabhängig voneinander $(C_1-C_6)$-Alkyl, $(C_2-C_4)$-Alkenyl, $(C_2-C_4)$-Alkinyl, $(C_3-C_6)$-Cycloalkyl, Phenyl, Benzyl, Wasserstoff, $(C_1-C_4)$-Alkoxy-$(C_1-C_4)$-alkyl, $(C_1-C_4)$Alkylthio-$(C_1-C_4)$-alkyl oder

$NR^5{}_2$ = Piperidino, Morpholino, Piperazino, Hydrazino oder Hydroxylamino oder einen Rest der Formel
$-A-SO_2R^4$, $-A-SOR^4$, $-A-SR^4$, $-A-P(O)R^4{}_2$ bedeutet.

In der Formel (I) bzw. (II) können Alkyl-, Alkoxy- und Alkylthioreste sowie die entsprechenden ungesättigten Reste jeweils geradkettig oder verzweigt sein. Halogen bedeutet Fluor, Chlor, Brom oder Jod, vorzugsweise Fluor, Chlor oder Brom, insbesondere Fluor oder Chlor.

Gegenstand der Erfindung sind insbesondere auch alle Stereoisomeren und deren Gemische, die von Formel (I) oder (II) umfaßt, jedoch nicht spezifisch definiert sind.

Gegenstand der vorliegenden Erfindung ist auch ein Verfahren zur Herstellung der neuen Verbindung der Formel (I) oder (II), dadurch gekennzeichnet, daß eine Verbindung der Formel (III)

$R^1 - SO_2 - CN$ (III),

worin $R^1$ die unter den Formeln (I) bzw. (II) genannte Bedeutung besitzt, mit einer Verbindung der Formel (IV)

$R^2$ - NH - $NH_2$ (IV),

worin $R^2$ die unter den Formeln (I) und (II) beschriebene Bedeutung hat, oder eines Salzes dieser Verbindung, in Gegenwart einer geeigneten Base, beispielsweise $NaHCO_3$, $K_2CO_3$ oder $NEt_3$ in einem polaren Lösungsmittel, beispielsweise $H_2O$, $CH_3OH$, $C_2H_5OH$ oder $CH_3CN$, umgesetzt wird.

Die Verbindungen der Formel (III) können nach verschiedenen lieteraturbekannten Methoden $a_1$, $a_2$, $a_3$, $a_4$ sowie nach einer neuen Methode $a_5$ gemäß folgendem Formelschema hergestellt werden:

$a_1$ 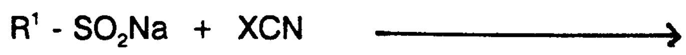 (III)

$a_2$ 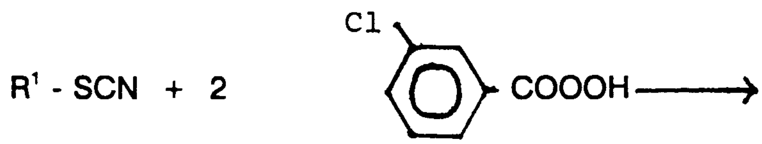 (III)

$a_3$ 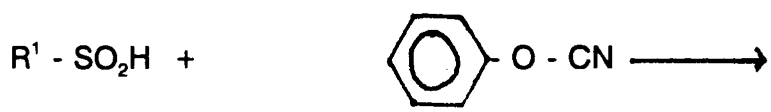 (III)

$a_4$ 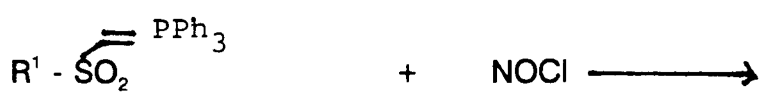 (III)

$a_5$ 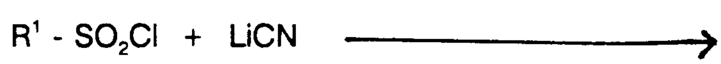 (III)

(V),

worin

$R^1$ = wie unter den Formeln (I) und (II) angegeben definiert sind und

X = Cl oder Br bedeutet.

Lit. ($a_1$-$a_4$): Tetrahedron Lett. Bd. 39, 3351 (1969).
J. Chem. Soc. Sect. D. Chem. Commun. 1969, 1187.
DE 2 248 940
US 3,755,306
DE 1 930 014
Organic Synthesis Vol. 57, 88 (1977)
J. Chem. Soc. Royal Netherlands 94 (1), 12 (1975)
J. Chem. Soc., Chem. Commun. 1968, 440.

Die erfindungsgemäßen Verbindungen der Formeln (I) und (II) zeichnen sich durch eine hervorragende pesticide, insbesondere fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl wirtschaftlich bedeutender, phytopathogener Pilze, wie z.B. Pyricularia oryzae, Phytophthora infestans, Plasmopara viticola, Leptosphaeria nodorum und verschiedene Rostpilze.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch Mittel, die die Verbindungen der Formel (I) oder (II) neben geeigneten Formulierungshilfsmitteln enthalten. Die erfindungsgemäßen Mittel enthalten die Wirkstoffe der Formel (I) oder (II) im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher in Frage:
Spritzpulver (WP), emulgierbare Konzentrate (EC), wäßrige Dispersionen auf Öl- oder Wasserbasis (SC), Suspoemulsionen (SC), Stäubemittel (DP), Beizmittel, Granulate in Form von wasserdispergierbaren Granulaten (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticide Formulations", Marcel Dekker N.Y., 2nd Ed. 1972-73; K. Martens, "Spray Drying Handbook", 3rd Ed. 1979, G. Goodwin Ltd., London.

Die notwendigen Formulierungshilfsmittel wie Inertmaterialien, Tenside, Lösungsmittel und weitere Zusatzstoffe sind ebenfalls bekannt und werden beispielsweise beschrieben in:
Watkins, "Handbook of Insecticide Dust Diluents and Carrier", 2nd Ed., Darland Books, Caldwell N.J.; H.v.Olphen, "Introduction to Clay Colloid Chemistry, 2nd Ed., J. Wiley & Sons, N.Y.; Mardsen, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1961; Schönfeldt, "Grenzflächenaktive Äthylenoxidaddukte", Wiss. Verlagsgesell., Stuttgart 1916; Winnacker-Küchler, "Chemische Technologie", Band 7, C. Hauser Verlag München, 1. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyethoxylierte Alkylphenole, polyethoxylierte Fettalkohole, Alkyl- oder Alkylphenylsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:
Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Sorbitanfettsäureester, Polyoxyethylensorbitan-Fettsäureester oder Polyoxyethylensorbitester.

Stäubemittel erhält man durch Vermahlen des Wirkstoffes mit fein verteilten festen Stoffen, z.B. Talkum, natürlichen Tonen wie Kaolin, Bentonit oder Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%. Bei Granulaten hängt der Wirkstoffgehalt zum Teil davon ab, ob die wirksame Verbindung flüssig oder fest vorliegt und welche Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formeln (I) und (II) kombiniert werden können, sind z.B. folgende Produkte zu nennen:
Anilazine, Benalaxyl, Benodanil, Benomyl, Binapacryl, Bitertanol, Buthiobat, Captafol, Captan, Carbendazim, Carboxin, CGD-94240 F, Chlobenzthiazone, Chlorthalonil, Cymoxanil, Cyproconazole, Cyprofuram, Dichlofluanid, Dichlomezin, Diclobutrazol, Diethofencarb, Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropomorph, Fentinacetat, Fentinhydroxid, Fluaziram, Fluobenzimine, Fluorimide, Flusilazole, Flutolanil, Flutriafol, Folpet, Fosetylaluminium, Fuberidazole, Furalaxyl, Furmecyclox, Guazatine, Hexaconazole, Imazalil, Iprobenfos, Iprodione, Isoprothiolane, Kupferverbindungen wie Cu-oxychlorid, Oxine-Cu, Cu-oxide, Mancozeb, Maneb, Mepronil, Metalaxyl, Methasulfocarb, Methfuroxam, Myclobutanil, Nabam, Nitrothalidopropyl, Nuarimol, Ofurace, Oxadixyl, Oxycarboxin, Penconazol, Pencycuron, PP 969, Probenazole, Probineb, Prochloraz, Procymidon, Propamocarb, Propiconazol, Prothiocarb, Pyracarbolid, Pyrifenox, Pyroquilon, Rabenzazole, Schwefel, Tebuconazole, Thiabendazole, Thiofanatemethal, Thiram, Tolclofos-methyl, Tolylfluanid, Triadimefon, Triadimenol, Tricyclazole, Tridemorph, Triflumizol, Triforine, Vinchlozolin, Zineb, Natrium-dodecylsulfonat, Natrium-dodecylsulfat, Natrium-C13/C15-alkoholethersulfonat, Natrium-cetostearylphosphatester, Dioctyl-natriumsulfosuccinat, Natrium-isopropylnaphthalinsulfonat, Natrium-methylenbisnaphthalinsulfonat, Cetyl-trimethylammoniumchlorid, Salze von langkettigen primären, sekundären oder tertiären Aminen, Alkyl-propylenamine, Lauryl-pyrimidiniumbromid, ethoxylierte quaternierte Fettamine, Alkyldimethyl-benzyl-ammoniumchlorid und 1-Hydroxyethyl-2-alkyl-imidazolin.

Die oben genannten Kombinationspartner stellen bekannte Wirkstoffe dar, die zum großen Teil in C.R. Worthing, S.B. Walker, The Pesticide Manual, 7. Aufl. (1983), British Crop Protection Council, beschrieben sind.

Darüberhinaus können die erfindungsgemäßen Wirkstoffe, insbesondere die der aufgeführten Beispiele, in ihren handelsüblichen Formulierungen sowie in den aus diesen Formulierungen bereiteten Anwendungsformen in Mischung mit anderen Wirkstoffe, wie Insektiziden, Lockstoffen, Sterilantien, Akariziden, Nematiziden, Fungiziden, wachstumsregulierenden Stoffe oder Herbiziden vorliegen. Zu den Insektiziden zählen beispielsweise Phosphorsäureester, Carbamate, Carbonsäureester, Formamide, Zinnverbindungen, durch Mikroorganismen hergestellte Stoffe u.a.. Bevorzugte Mischungspartner sind:

1. aus der Gruppe der Phosphorsäureester
Azinphos-ethyl, Azinphos-methyl, 1-(4-Chlorphenyl)-4-(O-ethyl, S-propyl)-phosphoryl-oxypyrazol (TIA-230), Chlorpyrifos, Coumaphos, Demeton, Demeton-S-methyl, Diazinon, Dichlorvos, Dimethoat, Ethoprophos, Etrimphos, Fenitrothion, Fenthion, Heptenophos, Parathion, Parathion-methyl, Phosalon, Pirimiphos-ethyl, Pirimiphos-methyl, Profenofos, Prothiofos, Sulprofos, Triazophos, Trichlorphon.

2. aus der Gruppe der Carbamate
Aldicarb, Bendiocarb, BPMC (2-(1-Methylpropyl)-phenylmethyl-carbamat), Butocarboxim, Butoxycarboxim, Carbaryl, Carbofuran, Carbosulfan, Cloethocarb, Isoprocarb, Methomyl, Oxamyl, Pirimicarb, Promecarb, Propoxur, Thiodicarb.

3. aus der Gruppe der Carbonsäureester
Allethrin, Alphamethrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)-cyclopropancarbonsäure-(alpha-cyano-3-phenyl-2-methyl-benzyl)ester (FMC 54800), Fenpropathrin, Fenfluthrin, Fenvalerat, Flucythrinate, Flumethrin, Fluvalinate, Permethrin, Resmethrin, Tralomethrin.

4. aus der Gruppe der Formamidine
Amitraz, Chlordimeform.

5. aus der Gruppe der Zinnverbindungen
Azocyclotin, Cyhexatin, Fenbutatinoxid.

6. sonstige

Abamektin, Bacillus thuringiensis, Bensultap, Binapacryl, Bromopropylate, Buprofecin, Camphechlor, Cartap, Chlorbenzilate, Chlorfluazuron, 2-(4-Chlorphenyl)-4,5-diphenylthiophen (UBI-T 930), Chlofentezine, Cyclopropancarbonsäure(2-naphthylmethyl)ester (Ro 12-0470), Cyromacin, DDT, Dicofol, N-(3,5-Dichlor-4-(1,1,2,2-tetrafluoroethoxy)-phenylamino)carbonyl)-2,6-difluor-benzamide (XRD 473), Diflubenzuron, N-(2,3-Dihydro-3-methyl-1,2-thiazol-2-ylidene)-2,4-xylidine, Dinobuton, Dinocap, Endosulfan, Fenoxycarb, Fenthiocarb, Flubenzimine, Flufenoxuron, Gamma-HCH, Hexythiazox, Hydramethylnon (AG 217 300), Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinan-3-yl-carbamaldehyde (WL 108 471), Propargite, Teflubenzuron, Tetradifon, Tetrasul, Thiocyclam, Triflumaron, Kernpolyeder- und Granuloseviren.

Der Wirkstoffgehalt der aus den handelsüblichen Formulierungen bereiteten Anwendungsformen kann in weiten Bereichen variieren, die Wirkstoffkonzentrationen der Anwendungsformen kann von 0,0001 bis zu 100 Gew.-% Wirkstoff, vorzugsweise zwischen 0,0001 bis 1 Gew.-% liegen. Die Anwendung geschieht in einer den Anwendungsformen angepaßten üblichen Weisen.

Nachfolgende Beispiele dienen zur Erläuterung der Erfindung.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 1 Gew.-Teil eines Sulfobernsteinsäurehalbesters, 2-Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

B. Chemische Beispiele

Beispiel 7 der Tabelle 1

n-Propylsulfonylcyanid

Zu einer Lösung von 56,4 g (0,447 mol) $Na_2SO_3$ und 75,1 g (0,894 mol) $NaHCO_3$ in 1000 ml Wasser werden bei 20 °C 63,7 g (0,447 mol) n-Propylsulfochlorid getropft. Nachdem eine klare Lösung entstanden ist werden bei 0 - 5 °C 55 g (0,894 mol) flüssiges ClCN schnell zugetropft und anschließend bei 20 °C 1 Stunde gerührt. Nach Extraktion mit $CH_2Cl_2$, Eindampfen und Destillation erhält man 51,1 g farbloses Öl vom Kp. 135 °C/10 Torr. $n^{22}_D$ = 1,4282

Beispiel 55 der Tabelle I

N-3,4-Dichlorphenyl-methylsulfonylformamidrazon

Zu einer Lösung von 5 g (0,048 mol) Methylsulfonylcyanid und 12,5 g (0,048 mol) 3,4-Dichlorphenylhydrazinhydrochlorid in 80 ml MeOH werden unter Eiskühlung 4,2 g (0,05 mol) $NaHCO_3$ zugegeben. Nach Beenden der Gasentwicklung wird auf Eiswaser gegeben und abgesaugt und anschließend durch Rühren mit n-Heptan gereinigt.

Ausbeute: 9,9 g vom Schmp.: 149-151 °C (Zers.)

Beispiel 28 der Tabelle I

N-3-Chlorbenzoyl-tosylformamidrazon

Zu einer Lösung von 5 g (0,028 mol) Tosylcyanid in 50 ml MeOH tropft man bei 0 °C 4,8 g (0,028 mol) 3-Chlorbenzoylhydrazid in 15 ml MeOH zu. Nach 2 Stunden Rühren bei 20 °C gibt man die Lösung auf Eiswasser, saugt ab und rührt zur Reinigung mit n-Heptan.
Ausbeute: 6,0 g vom Schmp.: 202-204 °C (Zers.)

In analoger Weise wurden die in der folgenden Tabelle I definierten Verbindungen der Formeln (I) und (II) (siehe Seite 1) erhalten.

## Tabelle I

| Bsp.-Nr. | $R^1$ | $R^2$ | Fp. (°C) (Zers.) |
|---|---|---|---|
| 1 | $C_6H_5$ | $CH_3$ | |
| 2 | " | $C_6H_5$ | |
| 3 | " | $4\text{-}ClC_6H_4$ | |
| 4 | " | $2,4\text{-}Cl(2)C_6H_3$ | 70 - 72 |
| 5 | " | $3,5\text{-}Cl(2)C_6H_3$ | |
| 6 | " | $3,4\text{-}Cl(2)C_6H_3$ | |
| 7 | " | $4\text{-}Br\text{-}C_6H_4$ | |
| 8 | " | $CH_2C_6H_5$ | |
| 9 | " | $C(O)C_6H_5$ | |
| 10 | $4\text{-}H_3CC(O)NHC_6H_4$ | $CH_3$ | 240 - 242 |
| 11 | " | $C_6H_5$ | |
| 12 | " | $4\text{-}ClC_6H_4$ | 155 - 157 |
| 13 | " | $2,4\text{-}Cl(2)C_6H_3$ | 186 - 188 |
| 14 | " | $3,5\text{-}Cl(2)C_6H_3$ | 178 - 180 |
| 15 | " | $3,4\text{-}Cl(2)C_6H_3$ | 186 - 188 |
| 16 | " | $4\text{-}Br\text{-}C_6H_4$ | |

| Bsp.-Nr. | $R^1$ | $R^2$ | Fp. (°C) (Zers.) |
|---|---|---|---|
| 17 | 4-$H_3CC(O)NHC_6H_4$ | $CH_2C_6H_5$ | 180 - 182 |
| 18 | " | $C(O)C_6H_5$ | |
| 19 | 4-$H_3CC_6H_4$ | $CH_3$ | 77 - 79 |
| 20 | " | $C_6H_5$ | 169 - 171 |
| 21 | " | 4-$ClC_6H_4$ | 141 - 142 |
| 22 | " | 2,4-Cl(2)$C_6H_3$ | 141 - 143 |
| 23 | " | 3,5-Cl(2)$C_6H_3$ | 148 - 150 |
| 24 | " | 3,4-Cl(2)$C_6H_3$ | 150 - 152 |
| 25 | " | 4-$BrC_6H_4$ | 157 - 159 |
| 26 | " | $CH_2C_6H_5$ | 94 - 96 |
| 27 | " | $C(O)C_6H_5$ | 129 - 131 |
| 28 | " | $C(O)C_6H_4$-3Cl | 202 - 204 |
| 29 | " | $C(O)CH_2C_6H_5$ | 131 - 133 |
| 30 | " | $C(O)CH_2CH_3$ | 126 - 128 |
| 31 | " | 3-$BrC_6H_4$ | 132 - 134 |
| 32 | 4-$tBuC_6H_4$ | $CH_3$ | |
| 33 | " | $C_6H_5$ | 135 - 137 |
| 34 | " | 4-$ClC_6H_4$ | |
| 35 | " | 2,4-Cl(2)$C_6H_3$ | 71 - 73 |
| 36 | " | 3,5-Cl(2)$C_6H_3$ | |
| 37 | " | 3,4-Cl(2)$C_6H_3$ | |
| 38 | " | 4-$BrC_6H_4$ | |

| Bsp.-Nr. | $R^1$ | $R^2$ | Fp. (°C) (Zers.) |
|---|---|---|---|
| 39 | 4-tBu$C_6H_4$ | $CH_2C_6H_5$ | |
| 40 | " | C(O)$C_6H_5$ | |
| 41 | 4-$F_3CC_6H_4$ | $CH_3$ | |
| 42 | " | $C_6H_5$ | |
| 43 | " | 4-Cl$C_6H_4$ | |
| 44 | " | 2,4-Cl(2)$C_6H_3$ | 135 - 137 |
| 45 | " | 3,5-Cl(2)$C_6H_3$ | |
| 46 | " | 3,4-Cl(2)$C_6H_3$ | |
| 47 | " | 4-Br$C_6H_4$ | |
| 48 | " | $CH_2C_6H_5$ | |
| 49 | " | C(O)$C_6H_5$ | |
| 50 | $CH_3$ | $CH_3$ | |
| 51 | " | $C_6H_5$ | |
| 52 | " | 4-Cl$C_6H_4$ | 156 - 158 |
| 53 | " | 2,4-Cl(2)$C_6H_3$ | 139 - 141 |
| 54 | " | 3,5-Cl(2)$C_6H_3$ | |
| 55 | " | 3,4-Cl(2)$C_6H_3$ | 149 - 151 |
| 56 | " | 4-Br$C_6H_4$ | |
| 57 | " | $CH_2C_6H_5$ | |
| 58 | " | C(O)$C_6H_5$ | |
| 59 | " | 2,6-Cl(2)-4-$CF_3C_6H_2$ | 135 - 137 |
| 60 | $C_2H_5$ | $CH_3$ | |

| Bsp.-Nr. | $R^1$ | $R^2$ | Fp. (°C) (Zers.) |
|---|---|---|---|
| 61 | $C_2H_5$ | $C_6H_5$ | |
| 62 | " | $4\text{-}ClC_6H_4$ | |
| 63 | " | $2,4\text{-}Cl(2)C_6H_3$ | 126 - 182 |
| 64 | " | $3,5\text{-}Cl(2)C_6H_3$ | |
| 65 | " | $3,4\text{-}Cl(2)C_6H_3$ | 140 - 142 |
| 66 | " | $4\text{-}BrC_6H_4$ | |
| 67 | " | $CH_2C_6H_5$ | |
| 68 | " | $C(O)C_6H_5$ | |
| 69 | $n\text{-}C_3H_7$ | $CH_3$ | |
| 70 | " | $C_6H_5$ | |
| 71 | " | $4\text{-}ClC_6H_4$ | |
| 72 | " | $2,4\text{-}Cl(2)C_6H_3$ | 142 - 144 |
| 73 | " | $3,5\text{-}Cl(2)C_6H_3$ | |
| 74 | " | $3,4\text{-}Cl(2)C_6H_3$ | 158 - 160 |
| 75 | " | $4\text{-}Br\text{-}C_6H_4$ | |
| 76 | " | $CH_2C_6H_5$ | |
| 77 | " | $C(O)C_6H_5$ | |
| 78 | $4\text{-}H_3COC_6H_4$ | $CH_3$ | |
| 79 | " | $C_6H_5$ | |
| 80 | " | $4\text{-}Cl\text{-}C_6H_4$ | |
| 81 | " | $2,4\text{-}Cl(2)C_6H_3$ | 140 - 142 |
| 82 | " | $3,5\text{-}Cl(2)C_6H_3$ | |

| Bsp.-Nr. | $R^1$ | $R^2$ | Fp. (°C) (Zers.) |
|---|---|---|---|
| 83 | 4-$H_3COC_6H_4$ | 3,4-Cl(2)$C_6H_3$ | |
| 84 | " | 4-Br $C_6H_4$ | |
| 85 | " | $CH_2C_6H_5$ | |
| 86 | " | C(O)$C_6H_5$ | |
| 87 | 4-$O_2NC_6H_4$ | $CH_3$ | 155 - 157 |
| 88 | " | $C_6H_5$ | |
| 89 | " | 4-$ClC_6H_4$ | |
| 90 | " | 2,4-Cl(2)$C_6H_3$ | |
| 91 | " | 3,5-Cl(2)$C_6H_3$ | |
| 92 | " | 3,4-Cl(2)$C_6H_3$ | |
| 93 | " | 4-$BrC_6H_4$ | |
| 94 | " | $CH_2C_6H_5$ | |
| 95 | " | C(O)$C_6H_5$ | |
| 96 | 3-Cl, 4-$H_3C(C_6H_3)$ | $CH_3$ | |
| 97 | " | $C_6H_5$ | |
| 98 | " | 4-$ClC_6H_4$ | |
| 99 | " | 2,4-Cl(2)$C_6H_3$ | 157 - 159 |
| 100 | " | 3,5-Cl(2)$C_6H_3$ | |
| 101 | " | 3,4-Cl(2)$C_6H_3$ | |
| 102 | " | 4-$BrC_6H_4$ | |
| 103 | " | $CH_2C_6H_5$ | |
| 104 | " | C(O)$C_6H_5$ | |

| Bsp.-Nr. | $R^1$ | $R^2$ | Fp. (°C) (Zers.) |
|---|---|---|---|
| 105 | $3\text{-}F_3CC_6H_4$ | $CH_3$ | |
| 106 | " | $C_6H_5$ | |
| 107 | " | $4\text{-}ClC_6H_4$ | |
| 108 | " | $2,4\text{-}Cl(2)C_6H_3$ | 197 - 199 |
| 109 | " | $3,5\text{-}Cl(2)C_6H_3$ | |
| 110 | " | $3,4\text{-}Cl(2)C_6H_3$ | |
| 111 | " | $4\text{-}BrC_6H_4$ | |
| 112 | " | $CH_2C_6H_5$ | |
| 113 | " | $C(O)C_6H_5$ | |
| 114 | $2,4,6\text{-}CH_3(3)C_6H_4$ | $CH_3$ | |
| 115 | " | $C_6H_5$ | |
| 116 | " | $4\text{-}ClC_6H_4$ | |
| 117 | " | $2,4\text{-}Cl(2)C_6H_3$ | 167 - 169 |
| 118 | " | $3,5\text{-}Cl(2)C_6H_3$ | |
| 119 | " | $3,4\text{-}Cl(2)C_6H_3$ | |
| 120 | " | $4\text{-}BrC_6H_4$ | |
| 121 | " | $CH_2C_6H_5$ | |
| 122 | " | $C(O)C_6H_5$ | |
| 123 | $C_6H_5CH_2$ | $CH_3$ | |
| 124 | " | $C_6H_5$ | |
| 125 | " | $4\text{-}Cl\ C_6H_4$ | |
| 126 | " | $2,4\text{-}Cl(2)C_6H_3$ | 112 - 114 |

| Bsp.-Nr. | $R^1$ | $R^2$ | Fp. (°C) (Zers.) |
|---|---|---|---|
| 127 | $C_6H_5CH_2$ | 3,5-Cl(2)$C_6H_3$ | |
| 128 | " | 3,4-Cl(2)$C_6H_3$ | |
| 129 | " | 4-Br$C_6H_4$ | |
| 130 | " | $CH_2C_6H_5$ | |
| 131 | " | C(O)$C_6H_5$ | |
| 132 | 4-F$C_6H_4$ | $CH_3$ | |
| 133 | " | $C_6H_5$ | |
| 134 | " | 4-Cl$C_6H_4$ | 146 - 148 |
| 135 | " | 2,4-Cl(2)-$C_6H_3$ | |
| 136 | " | 3,5-Cl(2)$C_6H_3$ | |
| 137 | " | 3,4-Cl(2)$C_6H_3$ | |
| 138 | " | 4-Br$C_6H_4$ | |
| 139 | " | $CH_2C_6H_5$ | |
| 140 | " | C(O)$C_6H_5$ | |
| 141 | 4-Br-$C_6H_4$ | $CH_3$ | |
| 142 | " | $C_6H_5$ | |
| 143 | " | 4-Cl$C_6H_4$ | 144 - 146 |
| 144 | " | 2,4-Cl(2)$C_6H_3$ | |
| 145 | " | 3,5-Cl(2)$C_6H_3$ | |
| 146 | " | 3,4-Cl(2)$C_6H_3$ | |
| 147 | " | 4-Br$C_6H_4$ | |
| 148 | " | $CH_2C_6H_5$ | |

| Bsp.-Nr. | $R^1$ | $R^2$ | Fp. (°C) (Zers.) |
|---|---|---|---|
| 149 | 4-Br-$C_6H_4$ | $C(O)C_6H_5$ | |
| 150 | 3,4-Cl(2)$C_6H_3$ | $CH_3$ | |
| 151 | " | $C_6H_5$ | |
| 152 | " | 4-Cl$C_6H_4$ | 142 - 144 |
| 153 | " | 2,4-Cl(2)$C_6H_3$ | |
| 154 | " | 3,5-Cl(2)$C_6H_3$ | |
| 155 | " | 3,4-Cl(2)$C_6H_3$ | 143 - 145 |
| 156 | " | 4-Br$C_6H_4$ | |
| 157 | " | $CH_2C_6H_5$ | |
| 158 | " | $C(O)C_6H_5$ | |
| 159 | 4-n$H_9C_4O$-$C_6H_4$ | $CH_3$ | |
| 160 | " | $C_6H_5$ | |
| 161 | " | 4-Cl$C_6H_5$ | 141 - 143 |
| 162 | " | 2,4-Cl(2)$C_6H_3$ | |
| 163 | " | 3,5-Cl(2)$C_6H_3$ | |
| 164 | " | 3,4-Cl(2)$C_6H_3$ | |
| 165 | " | 4-Br$C_6H_4$ | |
| 166 | " | $CH_2C_6H_5$ | |
| 167 | " | $C(O)C_6H_5$ | |
| 168 | 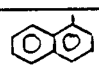 | $CH_3$ | |
| 169 | " | $C_6H_5$ | |
| 170 | " | 4-Cl$C_6H_4$ | |

| Bsp.-Nr. | $R^1$ | $R^2$ | Fp. (°C) (Zers.) |
|---|---|---|---|
| 171 | 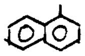 | 2,4-$Cl(2)C_6H_3$ | 136 - 138 |
| 172 | " | 3,5-$Cl(2)C_6H_3$ | |
| 173 | " | 3,4-$Cl(2)C_6H_3$ | |
| 174 | " | 4-$BrC_6H_4$ | |
| 175 | " | $CH_2C_6H_5$ | |
| 176 | " | $C(O)C_6H_5$ | |
| 177 | 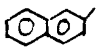 | $CH_3$ | |
| 178 | " | $C_6H_5$ | |
| 179 | " | 4-$ClC_6H_4$ | |
| 180 | " | 2,4-$Cl(2)C_6H_3$ | 135 - 137 |
| 181 | " | 3,5-$Cl(2)C_6H_3$ | |
| 182 | " | 3,4-$Cl(2)C_6H_3$ | |
| 183 | " | 4-$BrC_6H_4$ | |
| 184 | " | $CH_2C_6H_5$ | |
| 185 | " | $C(O)C_6H_5$ | |
| 186 | 4-$H_3CC(O)NCH_3C_6H_4$ | $CH_3$ | |
| 187 | " | $C_6H_5$ | |
| 188 | " | 4-$ClC_6H_4$ | |
| 189 | " | 2,4-$Cl(2)C_6H_3$ | 188 - 190 |
| 190 | " | 3,5-$Cl(2)C_6H_3$ | |
| 191 | " | 3,4-$Cl(2)C_6H_3$ | |
| 192 | " | 4-$BrC_6H_4$ | |

| Bsp.-Nr. | $R^1$ | $R^2$ | Fp. (°C) (Zers.) |
|---|---|---|---|
| 193 | $4\text{-}H_3CC(O)NCH_3C_6H_4$ | $CH_2C_6H_5$ | |
| 194 | " | $C(O)C_6H_5$ | |

C) Biologische Beispiele

Beispiel 1

Etwa 5 Wochen alte Reispflanzen der Sorte "Ballila" wurden mit den unten angegebenen Konzentrationen der erfindungsgemäßen Verbindungen behandelt. Nach Antrocknen des Spritzbelages wurden die Pflanzen mit einer Sporensuspension von Pyricularia oryzae gleichmäßig inokuliert und 48 Stunden in eine dunkel gehaltene Klimakammer mit einer Temperatur von 25 ° C und 100 % rel. Luftfeuchte gestellt. Danach wurden die Reispflanzen in einem Gewächshaus mit einer Temperatur von 25 ° C und 80 % rel. Luftfeuchte weiter kultiviert. Nach 5 Tagen erfolgte die Befallsauswertung. Der Befallsgrad wurde in befallener Blattfläche im Vergleich zu unbehandelten, infizierten Kontrollpflanzen ausgedrückt. Die Ergebnisse sind in Tabelle II zusammengestellt.

Tabelle II

| Verbindung gemäß Beispiel | mit Pyricularia oryzae befallene Blattflächen in % bei mg Wirkstoff/Liter Spritzbrühe | | |
|---|---|---|---|
| | 500 | 250 | 125 |
| 21 | 0 | 0 | 0 |
| 22 | 0 | 0 | 0 |
| 23 | 0 | 0 | 0 |
| 24 | 0 | 0 | 0 |
| 25 | 0 | 0 | 0 |
| 55 | 0 | 0 | 0 |
| 63 | 0 | 0 | 0 |
| 72 | 0 | 0 | 0 |
| 99 | 0 | 0 | 0 |
| 126 | 0 | 0 | 0 |
| 143 | 0 | 0 | 0 |
| unbehandelte, infizierte Pflanzen | 100 | | |

Beispiel 2

Tomatenpflanzen der Sorte "Rheinlands Ruhm" wurden im 3 - 4 Blattstadium mit wäßrigen Suspensionen der erfindungsgemäßen Verbindungen gleichmäßig tropfnaß benetzt.

Nach dem Antrocknen wurden die Pflanzen mit einer Zoosporangien-Suspension von Phytophthora infestans inokuliert und für 2 Tage unter optimalen Infektionsbedingungen in einer Klimakammer gehalten. Danach wurden die Pflanzen bis zur Symptomausprägung im Gewächshaus weiter kultiviert.

Die Befallsbonitur erfolgte ca. 1 Woche nach Inokulation. Der Befallsgrad der Pflanzen wurde in befallener Blattfläche, bezogen auf unbehandelte infizierte Pflanzen ausgedrückt und ist in Tabelle III wiedergegeben.

Tabelle III

| Verbindung gemäß Beispiel | mit Phytophthora infestans befallene Blattfläche in % bei mg Wirkstoff/Liter Spritzbrühe | | |
|---|---|---|---|
| | 500 | 250 | 125 |
| 25 | 0 | 0 | 0 |
| 55 | 0 | 0 | 0 |
| 63 | 0 | 0 | 0 |
| 72 | 0 | 0 | 0 |
| 143 | 0 | 0 | 0 |
| unbehandelte infizierte Pflanzen | 100 | | |

Beispiel 3

Weinsämlinge der Sorten "Riesling/Ehrenfelder" wurden ca. 6 Wochen nach der Aussaat mit wäßrigen Suspensionen der erfindungsgemäßen Verbindungen tropfnaß behandelt. Nach dem Antrocknen des Spritzbelages wurden die Pflanzen mit einer Zoosporangien-Suspension von Plasmopara viticola inokuliert und tropfnaß in eine Klimakammer mit 23 ° C und 80 - 90 % rel. Luftfeuchte gestellt.

Nach einer Inkubationszeit von 7 Tagen wurden die Pflanzen nochmals über Nacht in die Klimakammer gestellt, um die Sporulation des Pilzes anzuregen. Anschließend erfolgte die Befallsauswertung. Der Befallsgrad wurde in befallener Blattfläche im Vergleich zu den unbehandelten, infizierten Kontrollpflanzen ausgedrückt und ist in Tabelle IV wiedergegeben.

Tabelle IV

| Verbindung gemäß Beispiel | mit Plasmopara viticola befallene Blattfläche in % bei mg Wirkstoff/Liter Spritzbrühe | |
|---|---|---|
| | 500 | 250 |
| 21 | 0 | 0 |
| 55 | 0 | 0 |
| 63 | 0 | 0 |
| 72 | 0 | 0 |
| 108 | 0 | 0 |
| 143 | 0 | 0 |
| unbehandelte, infizierte Pflanzen | 100 | |

**Patentansprüche**

**1.** Sulfonylformamidrazone der allgemeinen Formel (I) und deren Tautomere der Formel (II)

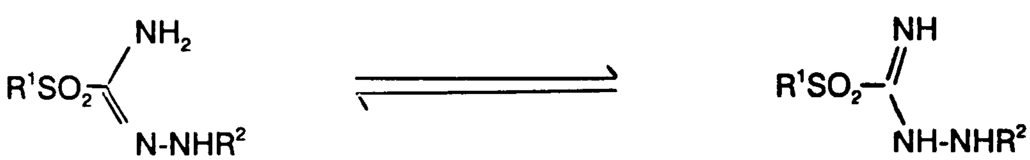

(I)

(II),

worin

$R^1$ = Alkyl, Alkenyl, Alkinyl, Cycloalkyl, Cycloalkenyl, wobei die 5 Gruppen unabhängig voneinander unsubstituiert oder ein- oder mehrfach durch die Reste Halogen, Cyano, Nitro, Mono- und Dialkylamino, Alkoxy, Alkenyloxy, Alkinyloxy, Alkylthio, Alkenylthio, Alkinylthio substituiert sein können oder einen Rest der Formel

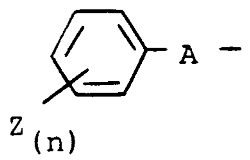

,

$R^2$ = Alkyl, Alkenyl, Alkinyl, Phenyl und Benzyl, wobei die 5 Gruppen unabhängig voneinander unsubstituiert oder ein- oder mehrfach durch die Reste Halogen, Cyano, Nitro, Mono- und Dialkylamino, Alkoxy, Haloalkyl substituiert sein können oder einen Rest der Formel

$-C(O)R^{2'}$, $-SO_2R^{2'}$, worin

$R^{2'}$ = Alkyl, Alkenyl, Alkinyl, Phenyl und Benzyl, wobei die 5 Gruppen unabhängig voneinander unsubstituiert oder ein- oder mehrfach durch die Reste Halogen, Cyano, Nitro, Mono- und Dialkylamino, Alkoxy, Haloalkyl substituiert sein können, bedeutet,

n = eine ganze Zahl von 0 - 5,

A = eine direkte Bindung, gesättigte oder ungesättigte, verzweigte oder unverzweigte Alkylkette,

Z = Wasserstoff, Halogen, Nitro, Cyano, Alkyl, Alkenyl, Alkinyl, Cycloalkyl, Cycloalkenyl, Alkoxy, Alkenyloxy, Alkinyloxy, Phenyl, Phenoxy, Haloalkyl, Haloalkoxy, Alkylthio, Haloalkylthio oder einen Rest der Formel

$-A-NR^3_2$ bedeuten, worin

$R^3$ = unabhängig voneinander Alkyl, Alkenyl, Alkinyl, Cycloalkyl, Phenyl, Benzyl, Alkoxy, Alkenyloxy, Alkinyloxy, Phenyloxy, Benzyloxy, Alkoxyalkyl, Alkylthioalkyl, Haloalkoxyalkyl, Haloalkylthioalkyl, Haloalkyl, Wasserstoff oder einen Rest der Formel

$-C(O)R^4$, $-SO_2R^4$, $-SOR^4$, $SR^4$ oder

$NR^3_2$ = Morpholino, Dialkylmorpholino, Piperidino, Piperazino, Hydrazino, Hydroxylamino, Phthalimido, Maleinimido, Dihydrophthalimido

oder einen Rest der Formel

$-A-C(O)R^4$ bedeutet, worin

$R^4$ = Wasserstoff, Alkyl, Alkenyl, Alkinyl, Cycloalkyl, Phenyl, Phenoxy, Phenylthio, OH, SH, Alkoxy, Alkenyloxy, Alkinyloxy, Alkylthio, Alkenylthio, Alkinylthio, Cycloalkylthio, Cycloalkoxy, Haloalkoxy, Haloalkylthio, Alkoxyalkyl, Alkylthioalkyl oder $-NR^5_2$ bedeutet, worin

$R^5$ = unabhängig voneinander Alkyl, Alkenyl, Alkinyl, Cycloalkyl, Phenyl, Benzyl, Wasserstoff, Alkoxyalkyl, Alkylthioalkyl oder

$NR^5_2$ = Piperidino, Morpholino, Piperazino, Hydrazino oder Hydroxylamino

oder einen Rest der Formel

$-A-SO_2R^4$, $-A-SOR^4$, $-A-SR^4$, $-A-P(O)R^4_2$ bedeutet.

**2.** Verbindungen der Formeln (I) und (II) gemäß Anspruch 1, dadurch gekennzeichnet, daß

$R^1$ = $(C_1-C_6)$-Alkyl, $(C_2-C_4)$-Alkenyl, $(C_2-C_4)$-Alkinyl, $(C_3-C_6)$-Cycloalkyl, $(C_3-C_6)$-Cycloalkenyl, wobei die 5 Gruppen unabhängig voneinander unsubstituiert oder ein- oder mehrfach durch die Reste Halogen, Cyano, Nitro, Mono- und Di-$(C_1-C_6)$-alkylamino, $(C_1-C_4)$-Alkoxy, $(C_2-C_4)$-Alkenyloxy, $(C_2-C_4)$-Alkinyloxy, $(C_1-C_4)$-Alkylthio, $(C_2-C_4)$-Alkenylthio, $(C_2-C_4)$-Alkinylthio substituiert sein können oder einen Rest der Formel

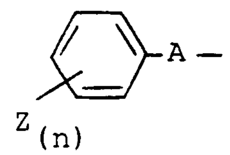

,

$R^2$ = ($C_1$-$C_6$)-Alkyl, ($C_2$-$C_4$)-Alkenyl, ($C_2$-$C_4$)-Alkinyl, Phenyl und Benzyl, wobei die 5 Gruppen unabhängig voneinander unsubstituiert oder ein- oder mehrfach durch die Reste Halogen, Cyano, Nitro, Mono- und Di-($C_1$-$C_4$)-alkylamino, ($C_1$-$C_4$)-Alkoxy, Halo-($C_1$-$C_4$)-alkyl substituiert sein können oder einen Rest der Formel

-C(O)$R^{2'}$, -$SO_2R^{2'}$, worin

$R^{2'}$ = ($C_1$-$C_6$)-Alkyl, ($C_2$-$C_4$)-Alkenyl, ($C_2$-$C_4$)-Alkinyl, Phenyl und Benzyl, wobei die 5 Gruppen unabhängig voneinander unsubstituiert oder ein- oder mehrfach durch die Reste Halogen, Cyano, Nitro, Mono- und Di-($C_1$-$C_4$)-alkylamino, ($C_1$-$C_4$)-Alkoxy, Halo-($C_1$-$C_4$)-alkyl substituiert sein können, bedeutet,

n = eine ganze Zahl von 0 - 5,

A = eine direkte Bindung, gesättigte oder ungesättigte, verzweigte oder unverzweigte Alkylkette,

Z = Wasserstoff, Halogen, Nitro, Cyano, ($C_1$-$C_6$)-Alkyl, ($C_2$-$C_4$)-Alkenyl, ($C_2$-$C_4$)-Alkinyl, ($C_3$-$C_6$)-Cycloalkyl, ($C_3$-$C_6$)-Cycloalkenyl, ($C_1$-$C_4$)-Alkoxy, ($C_2$-$C_4$)-Alkenyloxy, ($C_2$-$C_4$)-Alkinyloxy, Phenyl, Phenoxy, Halo-($C_1$-$C_4$)-alkyl, Halo-($C_1$-$C_4$)-alkoxy, ($C_1$-$C_4$)-Alkylthio, Halo-($C_1$-$C_4$)-alkylthio oder einen Rest der Formel

-A-$NR^3_2$ bedeuten, worin

$R^3$ = unabhängig voneinander ($C_1$-$C_6$)-Alkyl, ($C_2$-$C_4$)-Alkenyl, ($C_2$-$C_4$)-Alkinyl, ($C_3$-$C_6$)-Cycloalkyl, Phenyl, Benzyl, ($C_1$-$C_4$)-Alkoxy, ($C_2$-$C_4$)-Alkenyloxy, ($C_2$-$C_4$)-Alkinyloxy, Phenyloxy, Benzyloxy, ($C_1$-$C_4$)-Alkoxy-($C_1$-$C_4$)-alkyl, ($C_1$-$C_4$)-Alkylthio-($C_1$-$C_4$)-alkyl, Halo-($C_1$-$C_4$)-alkoxy-($C_1$-$C_4$)-alkyl, Halo-($C_1$-$C_4$)-alkylthio-($C_1$-$C_4$)-alkyl, Halo-($C_1$-$C_4$)-alkyl, Wasserstoff oder einen Rest der Formel

-C(O)$R^4$, -$SO_2R^4$, -$SOR^4$, $SR^4$ oder

$NR^3_2$ = Morpholino, Dialkylmorpholino, Piperidino, Piperazino, Hydrazino, Hydroxylamino, Phthalimido, Maleinimido, Dihydrophthalimido

oder einen Rest der Formel

-A-C(O)$R^4$ bedeutet, worin

$R^4$ = Wasserstoff, ($C_1$-$C_6$)-Alkyl, ($C_2$-$C_4$)-Alkenyl, ($C_2$-$C_4$)-Alkinyl, ($C_3$-$C_6$)-Cycloalkyl, Phenyl, Phenoxy, Phenylthio, OH, SH, ($C_1$-$C_4$)-Alkoxy, ($C_2$-$C_4$)-Alkyloxy, ($C_2$-$C_4$)-Alkinyloxy, ($C_1$-$C_4$)-Alkylthio, ($C_2$-$C_4$)-Alkenylthio, ($C_2$-$C_4$)-Alkinylthio, ($C_3$-$C_6$)-Cycloalkylthio, ($C_3$-$C_6$)-Cycloalkyloxy, Halo-($C_1$-$C_4$)-alkoxy, Halo-($C_1$-$C_4$)-alkylthio, ($C_1$-$C_4$)-Alkoxy-($C_1$-$C_4$)-alkyl, ($C_1$-$C_4$)-Alkylthio-($C_1$-$C_4$)-alkyl,
-$NR^5_2$ bedeutet, worin

$R^5$ = unabhängig voneinander ($C_1$-$C_6$)-Alkyl, ($C_2$-$C_4$)-Alkenyl, ($C_2$-$C_4$)-Alkinyl, ($C_3$-$C_6$)-Cycloalkyl, Phenyl, Benzyl, Wasserstoff, ($C_1$-$C_4$)-Alkoxy-($C_1$-$C_4$)-alkyl, ($C_1$-$C_4$)Alkylthio-($C_1$-$C_4$)-alkyl oder

$NR^5_2$ = Piperidino, Morpholino, Piperazino, Hydrazino oder Hydroxylamino

oder einen Rest der Formel

-A-$SO_2R^4$, -A-$SOR^4$, -A-$SR^4$, -A-P(O)$R^4_2$ bedeutet.

**3.** Verfahren zur Herstellung von Verbindungen der Formeln (I) und (II) gemäß Anspruch 1 oder 2, dadurch gekennzeichnet, daß eine Verbindung der Formel (III)

$R^1$ - $SO_2$ - CN (III),

worin $R^1$ die unter den Formeln (I) und (II) genannte Bedeutung besitzt, mit einer Verbindung der Formel (IV)

$R^2$ - NH - $NH_2$ (IV),

worin $R^2$ die unter den Formeln (I) und (II) beschriebene Bedeutung hat, oder eines Salzes dieser Verbindung, in Gegenwart einer geeigneten Base, in einem polaren Lösungsmittel umgesetzt wird.

**4.** Schädlingsbekämpfungsmittel, dadurch gekennzeichent, daß sie eine wirksame Menge einer Verbindung der Formel I oder II gemäß Anspruch 1 oder 2 und die üblichen Formulierungshilfsmittel enthalten.

**5.** Fungizide Mittel, dadurch gekennzeichent, daß sie eine wirksame Menge einer Verbindung der Formel I oder II gemäß Anspruch 1 oder 2 und die üblichen Formulierungshilfsmittel enthalten.

**6.** Verwendung von Verbindungen der Formel I oder II gemäß Anspruch 1 oder 2 zur Bekämpfung von Pflanzenschädlingen.

**7.** Verwendung von Verbindungen der Formel I oder II gemäß Anspruch 1 oder 2 zur Bekämpfung von Schadpilzen.

**8.** Verfahren zur Bekämpfung von Pflanzenschädlingen, dadurch gekennzeichnet, daß man auf diese oder die von ihnen befallenen Pflanzen, Flächen oder Substrate eine wirksame Menge einer Verbindung der Formel I oder II gemäß Anspruch 1 oder 2 appliziert.

**9.** Verfahren zur Bekämpung von Schadpilzen, dadurch gekennzeichnet, daß man auf diese oder die von ihnen befallenen Pflanzen, Flächen oder Substrate eine wirksame Menge einer Verbindung der Formel I oder II gemäß Anspruch 1 oder 2 appliziert.

Europäisches Patentamt

# EUROPÄISCHER RECHERCHENBERICHT

Nummer der Anmeldung

EP 92 10 9469

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | RECUEIL DES TRAVAUX CHIMIQUES DES PAYS-BAS. Bd. 94, Nr. 1, 1975, DEN HAAG NL Seiten 12 - 14; J. C. JAGT ET AL: 'NEUCLEOPHILIC ADDITIONS ON SULFONYL CYANIDES' * insgesamt * | 1-3 | C07C381/14 A01N47/42 |
| D,A | DE-A-2 248 940 (CHEMISCHE FABRIK VON HEYDEN) | | |
| D,A | US-A-3 755 306 (U. D. TREUNER) | | |

RECHERCHIERTE SACHGEBIETE (Int. Cl.5 )

C07C
A01N

Der vorliegende Recherchenbericht wurde für alle Patentansprüche erstellt

| Recherchenort | Abschlußdatum der Recherche | Prüfer |
|---|---|---|
| DEN HAAG | 17 AUGUST 1992 | ZAROKOSTAS K. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)